# EUROPÄISCHE PATENTSCHRIFT

(11) **EP 1 346 696 B1**
(45) Veröffentlichungstag und Bekanntmachung des Hinweises auf die Patenterteilung: **05.10.2005**
(21) Anmeldenummer: 03005280.7
(22) Anmeldetag: 11.03.2003
(51) Int. Cl.: A61B 17/72, A61B 17/17

(54) **Zielgerät für einen Verriegelungsnagel**
Aiming guide for use with intramedullary nail
Instrument de visée pour clou intramédullaire

(30) Priorität: 21.03.2002 DE 20204655 U
(43) Veröffentlichungstag der Anmeldung: 24.09.2003
(73) Patentinhaber: Stryker Trauma GmbH, 24232 Schönkirchen (DE)
(72) Erfinder: Zander, Nils, 24340 Eckernförde (DE); Cremer, Axel, 23795 Fahrenkrog (DE)
(74) Vertreter: Hauck, Graalfs, Wehnert, Döring, Siemons

(56) Entgegenhaltungen:
- EP-A- 0 550 814
- EP-A- 0 922 437
- EP-A- 1 090 596
- WO-A-99/35989
- US-A- 5 766 174

## Beschreibung

Die vorliegende Erfindung betrifft ein Zielgerät für einen Verriegelungsnagel.

Verriegelungsnägel zur Versorgung von Frakturen sind bekannt. Üblicherweise besitzen Verriegelungsnägel Querbohrungen am distalen Ende und mindestens eine Querbohrung am proximalen Ende. Durch die Querbohrungen werden Knochenschrauben hindurchgeführt, die an der gegenüberliegenden Seite in die Kortikalis eingeschraubt sind. Der Verriegelungsnagel wird durch die Knochenschrauben axial und gegen Drehung gesichert. Bei Suprakondylamägeln können auch Kondylenschrauben durch die Querbohrungen geführt werden.

Ein Problem bei der Verwendung von Verriegelungsnägeln besteht darin, im eingesetzten Zustand die Lage der Querbohrung zu identifizieren, um die Kortikalis von außen an der richtigen Stelle anzubohren. Es ist eine Reihe von Zielgeräten bekannt geworden, die mit Röntgenstrahlung arbeiten, um die Lage der Querbohrung relativ zu dem Zielgerät zu identifizieren. Es ist daher möglich, mit Hilfe des Zielgeräts und einer so genannten Bohr- oder Zielhülse den Knochen an der richtigen Stelle anzubohren. Die bekannten Zielgeräte werden zumeist fest mit dem Einschlagende des Nagels verbunden. Auf diese Weise lässt sich bereits annähernd genau die Lage der Querbohrung vorgeben. Allerdings ist zu berücksichtigen, dass durch die Krümmung des Knochens und durch die mögliche Torsion des Nagels beim Eintreiben die vermutete Lage der Querbohrung nicht mit der tatsächlichen übereinstimmt.

Nachteilig an dem Einsatz von mit Röntgenstrahlung arbeitenden Zielgeräten ist, dass sowohl der Operateur als auch der Patient einer Strahlenbelastung ausgesetzt ist. Es sind daher Zielgeräte ohne Durchleuchtungsmittel bekannt, die auf mechanischem Wege eine Lageidentifikation der Querbohrung erreichen. Da das Zielgerät mit dem Verriegelungsnagel verbunden werden muss, war bisher eine Lageidentifikation und Einführen von Knochenschrauben bei nahe zum Nagelende liegenden Querbohrungen nicht möglich. Die Querbohrungen müssen daher stets einen Mindestabstand von dem Ende des Verriegelungsnagels aufweisen.

Aus US 5,766,174 ist ein Zielgerät bekannt, eine Nagelhalteschraube mit einem zylindrischen Gewindekopf und eine Anschlußhülse, die einen in axialer Richtung vorstehenden Vorsprung besitzt. Zur Befestigung des Zielgeräts wird die Nagelhalteschraube durch die Anschlusshülse vorgeschoben und in dem Verriegelungsnagel verschraubt.

Der Erfindung liegt die Aufgabe zugrunde, ein Zielgerät für einen Verriegelungsnagel bereitzustellen, die mit einfachen Mitteln zuverlässig in der Lage sind, Querbohrungen nahe dem Ende des Verriegelungsnagels zum Einführen von Knochenschrauben oder Kondylenschrauben zu identifizieren.

Erfindungsgemäß wird die Aufgabe durch ein Zielgerät nach Anspruch 1 gelöst. Vorteilhafte Ausgestaltungen bilden den Gegenstand der Unteransprüche.

Erfindungsgemäß wird die Aufgabe durch ein Zielgerät mit den Merkmalen aus Anspruch 1 gelöst. Das Zielgerät besitzt eine Nagelhalteschraube sowie eine Anschlusshülse. Die Nagelhalteschraube ist an ihren zum Verriegelungsnagel weisenden Enden mit zwei in axialer Richtung vorstehenden Backen versehen. Der Zwischenraum zwischen den Backen gibt in einer vorbestimmten Position eine Querbohrung in dem Verriegelungsnagel frei. Die Nagelhalteschraube wird bei dem erfindungsgemäßen Zielgerät so weit in den Knochen eingesetzt, dass die am weitesten zum Ende liegende Querbohrung durch die Nagelhalteschraube nicht verdeckt wird, sondern durch den Zwischenraum zwischen den Backen frei bleibt. Die Nagelhalteschraube besitzt ebenfalls eine in axialer Richtung verlaufende Anlagefläche an der Außenseite. Die Anschlusshülse des Zielgeräts ist mit einem Mitnahmemittel versehen, das im aufgesetzten Zustand der Anschlusshülse mit der Anlagefläche der Nagelhalteschraube zusammenwirkt. Das Mitnahmemittel der Anschlusshülse liegt an der Anlagefläche an derart, dass die Nagelhalteschraube sich gemeinsam mit der Anschlusshülse dreht. Die Nagelhalteschraube wird also durch Drehen der Anschlusshülse in den Verriegelungsnagel geschraubt. Die Anschlusshülse ist ferner mit Mitteln zur Verdrehsicherung der Anschlusshülse gegenüber dem Verriegelungsnagel in der vorbestimmten Position der Nagelhalteschraube versehen. Bei dem erfindungsgemäßen Zielgerät ist die Anschlusshülse gegen eine Verdrehung gegenüber dem Verriegelungsnagel gesichert. Wird die Anschlusshülse in die verdrehgesicherte Position gebracht, so wird hierdurch die Nagelhalteschraube in ihre vorbestimmte Position gebracht, in der die Backen die Querbohrung freimachen.

Bevorzugt sind die Backen der Nagelhalteschraube mit einem Gewinde an der Außenseite versehen. Der Grund zwischen den Backen ist gerundet und die freien Enden der Backen sind abgerundet. Die Nagelhalteschraube wird mit dem distalen Ende des Verriegelungsnagels verschraubt, wobei der Zwischenraum zwischen den Backen die Querbohrung freigibt. Die Backen sind derart geformt, dass ein nach dem Bohren eingebrachter Kirschnerdraht durch die Backen bei einem Entfernen der Nagelhalteschraube in der Bohrung verbleiben kann und lediglich an deren Rand geschoben wird.

Bevorzugt besitzt die Nagelhalteschraube an dem zum Verriegelungsnagel weisenden Ende einen umlaufenden Vorsprung. Zwischen dem Abschnitt mit den Backen und dem Vorsprung ist eine umlaufende Nut vorgesehen. Die Anschlusshülse besitzt stirnseitig drei Vorsprünge, die paarweise Winkel von 90° oder 180° miteinander einschließen und im aufgesetzten Zustand in die Ausnehmungen an dem Verriegelungsnagel eingreifen.

Die Anlagefläche ist bevorzugt als eine Abflachung oder als eine Nut ausgebildet. Es ist jedoch auch denkbar, den Schaft der Nagelhalteschraube in einem Bereich mit mehreren ebenen Flächen zu versehen.

Bevorzugt ist als Mitnahmemittel der Anschlusshülse eine Bohrung und ein darin eingesetzter Stift vorgesehen. Der in die Bohrung eingesetzte Stift erstreckt sich senkrecht und beabstandet zu der Mittellängsachse der Anschlusshülse. Beide Enden des Stifts sind im eingesetzten Zustand in der Wand der Anschlusshülse gehalten. Die Anschlusshülse ist ferner mit Orientierungsausnehmungen für einen Griffabschnitt des Zielgeräts versehen. Der Griffabschnitt ist bevorzugt mit Mitteln versehen zur Festlegung axial und in Drehrichtung relativ zu den Orientierungsausnehmungen. Hierzu besitzt der Griffabschnitt bevorzugt eine Bohrung, in die ein federgespannter Stift vorsteht, der in eine der Orientierungsausnehmungen auf der Anschlusshülse eingreift.

Der Griffabschnitt ist ferner mit einer Arretierungsschraube versehen, die in ihrer eingeschraubten Position den Stift gegen die Orientierungsausnehmung der Anschlusshülse presst.

Zur Festlegung in der Anschlusshülse in axialer Richtung ist die Nagelhalteschraube an ihrem vom Verriegelungsnagel fortweisenden Ende mit einem Gewindeabschnitt versehen, der im aufgesetzten Zustand der Anschlusshülse aus dieser hervorsteht. Zur Festlegung der Anschlusshülse in axialer Richtung wird auf den Gewindeabschnitt eine Mutter geschraubt, die die Anschlusshülse gegen den Verriegelungsnagel drückt.

Die vorliegende Erfindung wird nachfolgend anhand eines Ausführungsbeispiels näher erläutert. Es zeigt:
Fig. 1 das distale Ende eines Verriegelungsnagels,
Fig. 2 eine Nagelhalteschraube in einer ersten Seitenansicht,
Fig. 3 die Nagelhalteschraube aus Fig. 2 in einer zweiten Seitenansicht,
Fig. 4 einen Schnitt entlang der Linie 4-4 aus Fig. 3,
Fig. 5 eine Anschlusshülse aus einer ersten Seitenansicht,
Fig. 6 die Anschlusshülse aus Fig. 5 aus einer zweiten Ansicht,
Fig. 7 die Anschlusshülse aus Fig. 5 aus einer dritten Ansicht,
Fig. 8 einen Schnitt entlang der Linie 8-8 aus Fig. 5,
Fig. 9 eine auseinandergezogene Ansicht von Nagelhalteschraube und Anschlusshülse,
Fig. 10 einen Schnitt entlang der Linie 10-10 in Fig. 9,
Fig. 11 eine auseinandergezogene Darstellung von Nagelhalteschraube und Anschlusshülse in der perspektivischen Ansicht,
Fig. 12 eine Schnittdarstellung von einem auf den Verriegelungsnagel gesetzten Zielgerät,
Fig. 13 ein Griffteil des Zielgeräts aus Fig. 12 und
Fig. 14 eine Griffteilhalterung auf der Anschlusshülse.

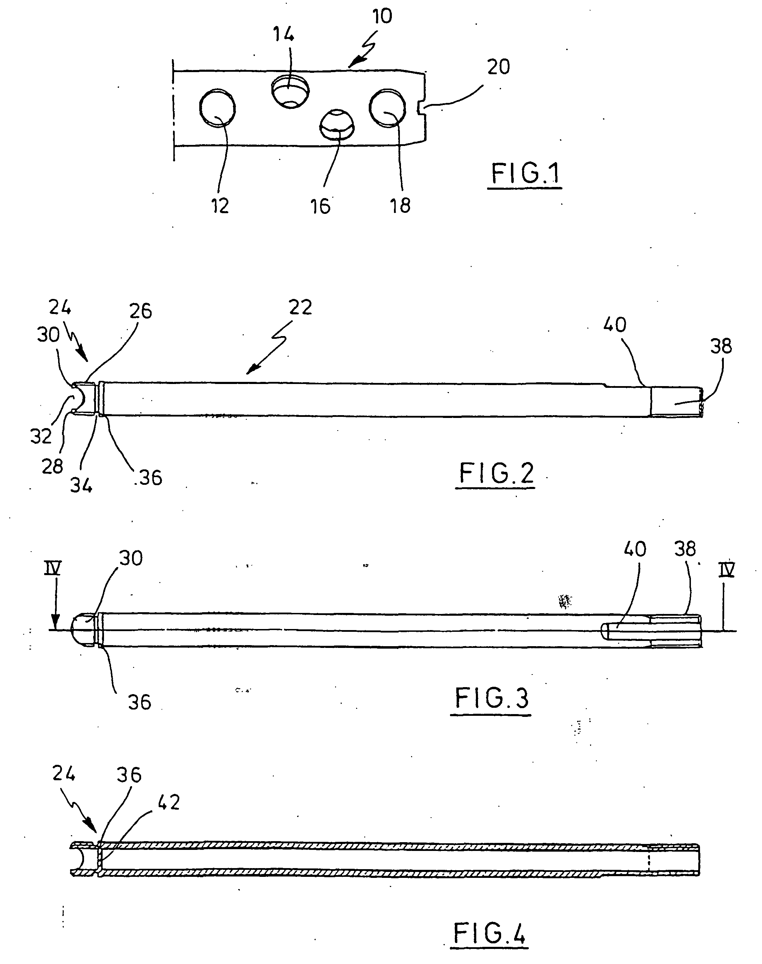

Fig. 1 zeigt das distale Ende eines Suprakondylarnagels 10. Der Verriegelungsnagel ist mit vier Querbohrungen 12 bis 18 versehen, die zur Aufnahme von Knochenschrauben und/oder Kondylarschrauben zur axialen und radialen Festlegung des Verriegelungsnagels in dem Knochen dienen. Stirnseitig ist der Verriegelungsnagel mit Ausnehmungen versehen, von denen zwei in einem 180°-Winkel voneinander beabstandet in Fig. 1 sichtbar sind. Die Ausnehmung 20 besitzt eine rechteckige Form. Bei dem dargestellten Verriegelungsnagel besitzt die Querbohrung 18 einen Abstand von dem freien Ende des Nagels von 6 mm. Nicht sichtbar in Fig. 1 ist ein Innengewinde, das in dem Verriegelungsnagel vorgesehen ist und sich vom Ende des Verriegelungsnagels über den Beginn der Querbohrung 18 hinaus erstreckt.

Fig. 2 zeigt eine Nagelhalteschraube 22. Das zur Verbindung mit dem Verriegelungsnagel vorgesehene Ende 24 besitzt ein Außengewinde 26, das dem oben genannten Innengewinde des Verriegelungsnagels korrespondiert. Das Ende 24 besitzt zwei Backen 28 und 30, die eine Ausnehmung 32 begrenzen. Die Backen 28, 30 stehen in axialer Richtung vor und bilden ein u-förmiges Ende der Nagelhalteschraube. An den Gewindeabschnitt anschließend ist eine umlaufende Nut 34 vorgesehen, der ein umlaufender Vorsprung 36 folgt.

Das gegenüberliegende Ende der Nagelhalteschraube 22 ist mit einem Gewindeabschnitt 38 versehen. Das Gewinde 38 ist im Bereich der Abflachung 40 unterbrochen. Bei dem Gewinde 38 liegt also ein Teilgewinde vor. Fig. 3 zeigt eine Draufsicht auf die Abflachung 40. Ein Schnitt entlang der Linie 4-4 aus Fig. 3 ist in Fig. 4 dargestellt. In der Schnittdarstellung 4 ist ersichtlich, dass der Bereich 24 gegenüber dem Schaft der Nagelhalteschraube durch eine Trennwand 42 getrennt ist.

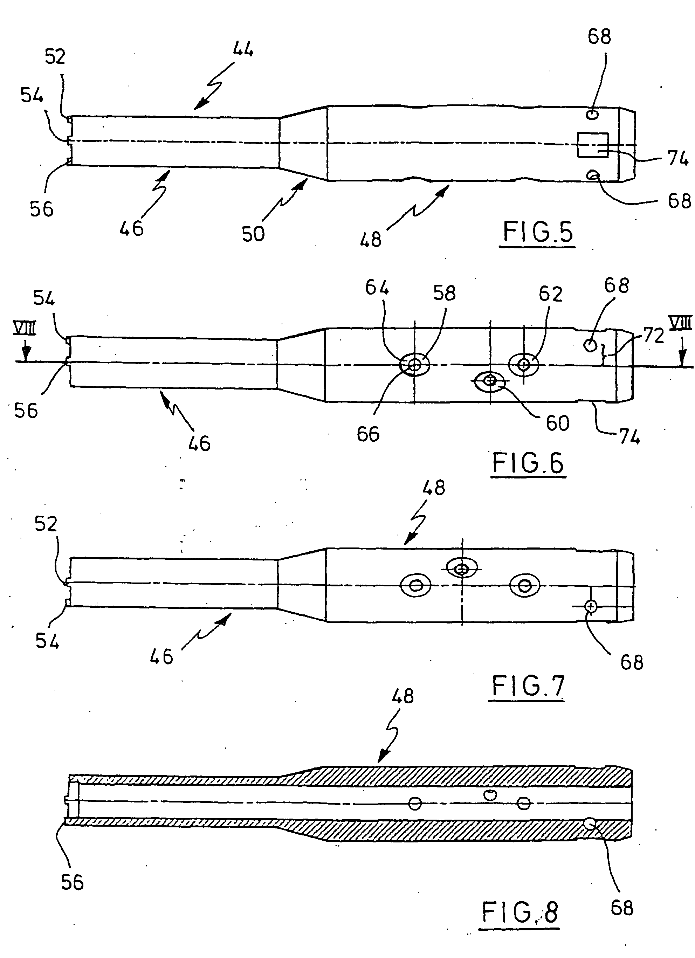

Die erfindungsgemäße Anschlusshülse wird mit Bezug auf die Figuren 5 bis 8 näher beschrieben. Die Anschlusshülse 44 besitzt einen zylindrischen Schaft mit einem ersten Abschnitt 46 und einem zweiten Abschnitt 48, der einen größeren Radius als der erste Abschnitt besitzt. Der Übergangsbereich 50 zwischen erstem und zweitem Abschnitt ist konisch ausgestaltet. Der Innendurchmesser der Anschlusshülse entspricht dem Außendurchmesser der Nagelhalteschraube, sodass diese auf die Nagelhalteschraube geschoben werden kann.

Das Ende des ersten Abschnitts 46 ist mit drei Vorsprüngen 52 bis 56 versehen. Die Vorsprünge 52 bis 56 korrespondieren mit Ausnehmungen 20 in dem Verriegelungsnagel. Angeordnet sind die Vorsprünge 52 bis 56 in einem Halbkreissegment, sodass die Vorsprünge 52 und 54 einen 90°-Winkel, die Vorsprünge 54 und 56 ebenfalls einen 90°-Winkel und die Vorsprünge 56 und 52 einen 180°-Winkel einschließen.

In dem zweiten Abschnitt 48 sind Orientierungsausnehmungen 58 bis 62 vorgesehen. Die Orientierungsausnehmungen besitzen einen trichterförmigen Bereich 64 mit einer zentralen Durchgangsbohrung 66.

Am Ende des zweiten Abschnitts 48 ist eine Durchgangsbohrung 68 vorgesehen. Die Durchgangsbohrung 68 ist um den Abstand 70 von der Mittellängsachse der Hülse beabstandet und verläuft senkrecht zu dieser. Ein Stift 76 wird in die Durchgangsbohrung eingepresst und verengt deren Durchmesser. Der Querschnitt der Hülse mit eingesetztem Stift entspricht der Nagelhalteschraube.

In dem zweiten Abschnitt 48 sind ebenfalls Schlüsselflächen 74 für einen Maulschlüssel oder dergleichen vorgesehen.

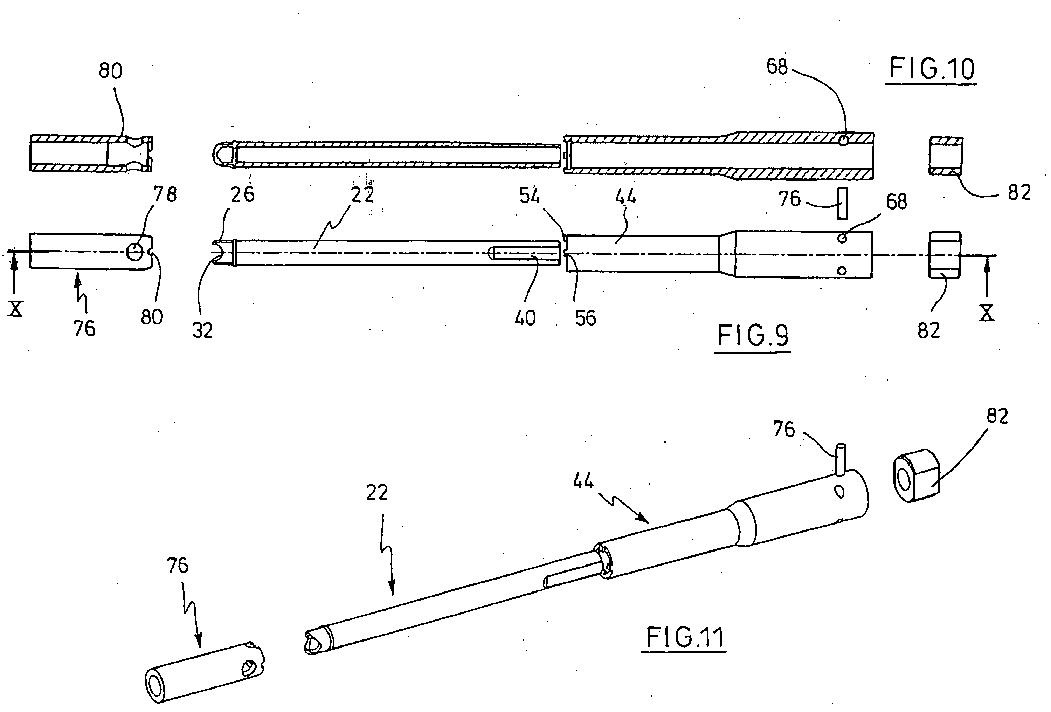

Fig. 9 zeigt ein Ende eines Verriegelungsnagels 76 mit einer Querbohrung 78. Das Ende des Verriegelungsnagels ist mit einem Innengewinde 80 versehen, wie aus der Schnittdarstellung 10 ersichtlich ist. Die Nagelhalteschraube 22 wird mit ihrem Außengewinde 26 in den Verriegelungsnagel eingeschraubt. In die Bohrung 68 der Anschlusshülse 44 wird ein Stift 76 eingepresst. Durch den Stift 76 wird der Innendurchmesser der Hülse 44 verengt, wie in Fig. 10 ersichtlich. Wird die Anschlusshülse 44 mit dem eingepressten Stift 76 auf die Nagelhalteschraube 22 aufgeschoben, so ist ein Aufschieben nur möglich, wenn der Stift 76 an der Abflachung 40 anliegt. Die so aufgeschraubte Haltehülse 44 wird an der Stirnseite des Verriegelungsnagels mit den Vorsprüngen 54 und 56 entsprechend zu den Ausnehmungen 80 ausgerichtet. Hierbei korrespondiert jedem Vorsprung eine Ausnehmung. Durch das Zusammenwirken von Vorsprung und Ausnehmung kann die Anschlusshülse und damit auch die Nagelhalteschraube lediglich in einer Position an dem Verriegelungsnagel festgelegt werden. In dieser Position gibt der Zwischenraum 32 der Nagelhalteschraube die Querbohrung 78 frei.

Axial wird die Anschlusshülse 44 durch eine Mutter 82 gesichert, die auf dem Gewindeabschnitt 38 der Nagelhalteschraube aufgeschraubt wird.

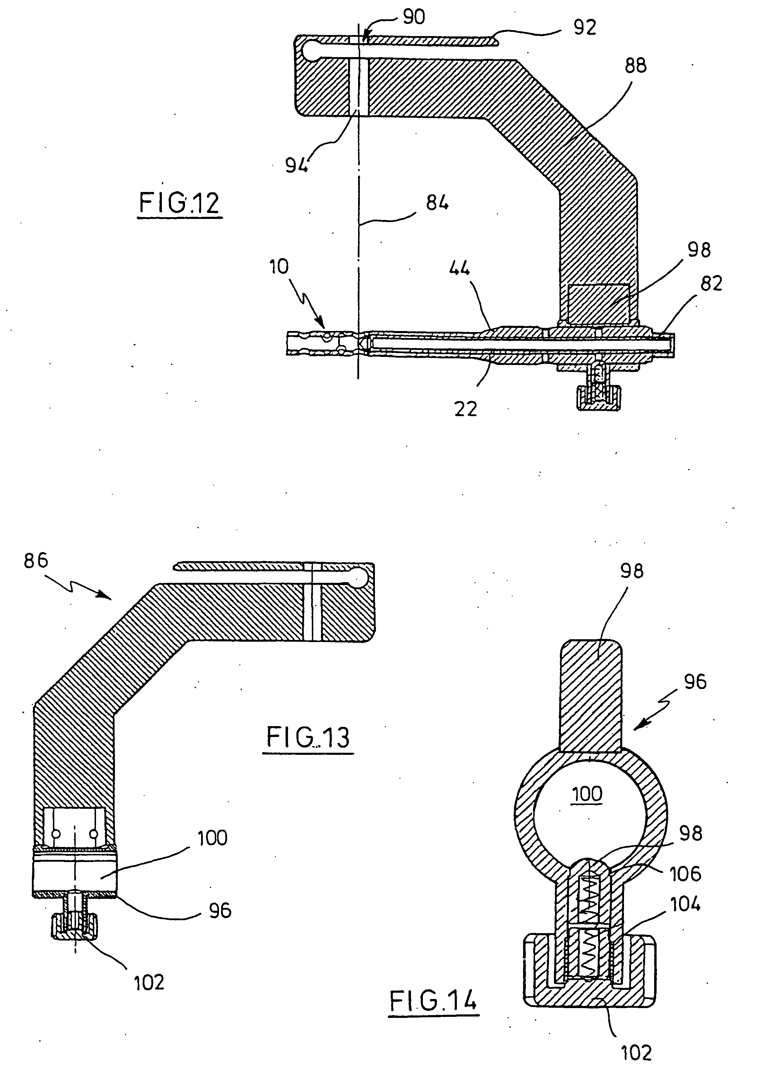

Fig. 12 zeigt einen Querschnitt durch das gesamte Zielgerät in seiner aufgesetzten Position. An den Verriegelungsnagel 10 ist die Nagelhalteschraube 22 geschraubt, auf die die Anschlusshülse 44 gesetzt ist. Die Anschlusshülse 44 ist in axialer Richtung durch die Mutter 82 gesichert. Die Backen 28, 30 der Nagelhalteschraube 22 sind so ausgerichtet, dass die am nächsten zu dem Ende liegende Querbohrung entlang der gedachten Linie 84 freigegeben ist. Auf den zweiten Abschnitt 48 der Anschlusshülse ist ein Griffteil 86 des Zielgeräts aufgesetzt. Das Griffteil 86 ist axial und in Verdrehrichtung gesichert. Das Griffteil 86 besitzt einen ersten Abschnitt 88, der als Handgriff ausgebildet ist und eine Zieleinrichtung 90 für eine Ziel- oder Bohrungshülse besitzt. Durch Andrücken der Lasche 92 wird eine in die Zielbohrung 94 eingesetzte Hülse in ihrer Position gesichert.

Über eine Zapfenverbindung ist der erste Abschnitt 88 mit einer Feststelleinrichtung 96 verbunden. Die Feststelleinrichtung 96 besitzt einen Zapfen 98, über den das Griffteil verbunden wird. Die Feststelleinrichtung 96 besitzt eine zentrale Bohrung 100, deren Innendurchmesser dem Außendurchmesser der Hülse im zweiten Abschnitt entspricht. In die Bohrung 100 hinein ragt ein federvorgespannter Stift 98, dessen in die Bohrung 100 hineinragende Form bildet gemeinsam mit den trichterförmigen Vertiefungen der Orientierungsausnehmungen eine Schnappverbindung. Der Stift 98 schnappt in eine der Markierungsausnehmungen 60 bis 64 auf der Anschlusshülse ein. Ist der Stift 98 nicht in eine Markierungsausnehmung eingeschnappt, so wird er gegen die Federspannung nach außen gedrückt und das Feststellteil 96 kann auf der Anschlusshülse bewegt werden.

Der eingeschnappte Stift 98 wird über eine Arretierschraube 102 gesichert. Die Arretierschraube 102 besitzt einen Schaft 104, der über ein Gewinde in die Bohrung des Stifts 98 eingeschraubt wird. In seiner eingeschraubten Position liegt der Schaft 104 an dem Stift 98 an und presst diesen gegen die Anschlusshülse.

Der Stift 98 ist durch einen umlaufenden Absatz 106, der mit einem umlaufenden Vorsprung der Feststelleinrichtung 96 zusammenwirkt, gegen zu weites Hineinschieben in die Öffnung 100 gesichert.

Zur besseren Orientierung für den Operateur können die einzelnen Markierungsausnehmungen gekennzeichnet sein, damit erkennbar ist, auf welche der Querbohrungen die Zieleinrichtung 90 gerichtet ist. Ist das Zielgerät ausgerichtet, wird seine Position durch die Arretierschraube 102 gesichert.

Das Einsetzen einer Kondylenschraube in die am nächsten zum Rand liegende Querbohrung erfolgt über einen Kirschnerdraht, der beispielsweise einen Durchmesser von 1,8 mm besitzt. Nach dem Bohren, mit beispielsweise einem Bohrer von 5 mm Durchmesser wird der Kirschnerdraht eingebracht und das Zielgerät kann entfernt werden. Würde die Kondylenschraube eingesetzt, bevor das Zielgerät entfernt ist, könnte die Nagelhalteschraube nicht mehr von dem Verriegelungsnagel abgeschraubt werden, da deren Backen durch die Kondylenschraube blockiert sind. Jedoch sind die Backen derart abgerundet, dass der Kirschnerdraht in der Querbohrung verdrängt wird und die Nagelhalteschraube abgeschraubt werden kann. Nachfolgend wird die Kondylenschraube über den Kirschnerdraht in die Querbohrung eingeführt und verschraubt.

## Patentansprüche

1. Zielgerät für einen Vernegelungsnagel, mit
- einer Nagelhalteschraube (22), die an ihrem zur Befestigung mit dem Verriegelungsnagel vorgesehenen Ende zwei in axialer Richtung vorstehende Backen (28, 30), deren Zwischenraum (32) in einer vorbestimmten Position eine Querbohrung in dem Verriegelungsnagel freigibt, und eine in axialer Richtung verlaufende Anlagefläche (40) an der Außenseite der Nagelhalteschraube besitzt, und
- einer Anschlusshülse (44), die
-- ein Mitnahmemittel (68, 70) aufweist, das im auf die Nagelhalteschraube gesetzten Zustand mit der Anlagefläche (40) zusammenwirkt derart, dass die Nagelhalteschraube gemeinsam mit der Anschlusshülse dreht, und
-- Mittel (52, 54, 56) zur Verdrehsicherung der Anschlusshülse gegenüber dem Verriegelungsnagel in der vorbestimmten Position der Nagelhalteschraube besitzt.

2. Zielgerät nach Anspruch 1, **dadurch gekennzeichnet, dass** die Backen (28, 30) der Nagelhalteschraube ein Gewinde (26) an der Außenseite besitzen zur Verbindung mit einem Innengewinde an dem Verriegelungsnagel.

3. Zielgerät nach Anspruch 1 oder 2, **dadurch gekennzeichnet, dass** der Grund zwischen den Backen gerundet ist.

4. Zielgerät nach einem der Ansprüche 1 bis 3, **dadurch gekennzeichnet, dass** die freien Enden der Backen abgerundet sind.

5. Zielgerät nach einem der Ansprüche 1 bis 4, **dadurch gekennzeichnet, dass** die Nagelhalteschraube an dem zum Verriegelungsnagel weisenden Ende einen umlaufenden Vorsprung (36) besitzt.

6. Zielgerät nach Anspruch 7, **dadurch gekennzeichnet, dass** zwischen den Backen und dem Vorsprung eine umlaufende Nut (34) vorgesehen ist.

7. Zielgerät nach einem der Ansprüche 1 bis 6, **dadurch gekennzeichnet, dass** die Anschlusshülse stirnseitig drei Vorsprünge besitzt, die paarweise Winkel von 90° oder 180° miteinander einschließen.

8. Zielgerät nach einem der Ansprüche 1 bis 7, **dadurch gekennzeichnet, dass** die Mittel zur Verdrehsicherung der Anschlusshülse stirnseitig Vorsprünge und/oder Ausnehmungen aufweisen.

9. Zielgerät nach einem der Ansprüche 1 bis 8, **dadurch gekennzeichnet, dass** das Mitnahmemittel der Anschlusshülse eine Bohrung (68) und einen darin einsetzbaren Stift (76) aufweist.

10. Zielgerät nach Anspruch 9, **dadurch gekennzeichnet, dass** die Bohrung (68) beabstandet zu einer Mittellängsachse der Anschlusshülse verläuft und der Stift (76) mit seiner Mantelfläche an der Anlagefläche anliegt.

11. Zielgerät nach einem der Ansprüche 1 bis 10, **dadurch gekennzeichnet, dass** die Anschlusshülse Orientierungsausnehmungen (58, 60, 62) für einen Griffabschnitt besitzt.

12. Zielgerät nach Anspruch 11, **dadurch gekennzeichnet, dass** der Griffabschnitt Mittel zur Festlegung axial und in Drehrichtung relativ zu den Orientierungsausnehmungen besitzt.

13. Zielgerät nach Anspruch 12, **dadurch gekennzeichnet, dass** der Griffabschnitt (88) eine Bohrung zur Aufnahme der Anschlusshülse besitzt, in die ein federvorgespannter Stift (98) zum Eingriff in eine der Orientierungsausnehmungen vorsteht.

14. Zielgerät nach Anspruch 13, **dadurch gekennzeichnet, dass** der Griffabschnitt eine Arretierschraube (102) besitzt, die in eingeschraubter Position den Stift (98) in eine der Orientierungsausnehmungen presst.

15. Zielgerät nach einem der Ansprüche 1 bis 14, **dadurch gekennzeichnet, dass** die Nagelhalteschraube an ihrem von dem Verriegelungsnagel fortweisenden Ende einen Gewindeabschnitt aufweist, der in aufgesetztem Zustand der Anschlusshülse aus dieser hervorsteht.

## Claims

1. Aiming guide for a locking nail, with
- a nail retention screw (22) which, at its end provided for fastening to the locking nail, has two cheeks (28, 30), projecting in the axial direction, whose intermediate space (32) opens up a cross-bore in the locking nail in a predetermined position, and has a bearing surface (40) extending in the axial direction on the outer face of the nail retention screw and
- a joining sleeve (44) which
- comprises a drive means (68, 70) which, when placed on the nail retention screw, co-operates with the bearing surface (40) such that the nail retention screw rotates together with the joining sleeve and
- has means (52, 54, 56) for anti-rotationally securing the joining sleeve relative to the locking nail in the predetermined position of the nail retention screw.

2. Aiming guide according to claim 1, **characterised in that** the cheeks (28, 30) of the nail retention screw have a thread (26) on the outer face for connecting to a female thread on the locking nail.

3. Aiming guide according to claim 1 or 2, **characterised in that** the base between the cheeks is rounded.

4. Aiming guide according to any one of claims 1 to 3, **characterised in that** the free ends of the cheeks are rounded.

5. Aiming guide according to any one of claims 1 to 4, **characterised in that** the nail retention screw has a circumferential projection (36) on the end facing the locking nail.

6. Aiming guide according to claim 7, **characterised in that** a peripheral groove (34) is provided between the cheeks and the projection.

7. Aiming guide according to any one of claims 1 to 6, **characterised in that** the joining sleeve has three projections on the front face which enclose, in pairs, an angle of 90 DEG or 180 DEG with one another.

8. Aiming guide according to any one of claims 1 to 7, **characterised in that** the means for anti-rotationally securing the joining sleeve comprise projections and/or recesses on the front face.

9. Aiming guide according to any one of claims 1 to 8, **characterised in that** the drive means of the joining sleeve comprises a bore (68) and a pin (76) which can be inserted therein.

10. Aiming guide according to claim 9, **characterised in that** the bore (68) extends at a distance from a central longitudinal axis of the joining sleeve and the pin (76) bears against the bearing surface with its cylindrical surface.

11. Aiming guide according to any one of claims 1 to 10, **characterised in that** the joining sleeve has orientation recesses (58, 60, 62) for a handle portion.

12. Aiming guide according to claim 11, **characterised in that** the handle portion has axial and rotational securing means relative to the orientation recesses.

13. Aiming guide according to claim 12, **characterised in that** the handle portion (88) has a bore for receiving the joining sleeve into which a spring-biased stud (98) projects for engaging in one of the orientation recesses.

14. Aiming guide according to claim 13, **characterised in that** the handle portion has a locking screw (102) which, in the screwed-in position, presses the stud (98) into one of the orientation recesses.

15. Aiming guide according to any one of claims 1 to 14, **characterised in that** at its end facing away from the locking nail, the nail retention screw comprises a threaded portion which, when the joining sleeve is in place, projects therefrom.

## Revendications

1. Instrument de visée pour clou intramédullaire, avec
- une vis de retenue de clou (22) qui, à son extrémité prévue pour la fixation avec le clou intramédullaire, possède deux joues (28, 30) faisant saillie dans la direction axiale et dont l'espace intermédiaire (32) libère, dans une position prédéfinie, un alésage transversal dans le clou intramédullaire, et une surface d'appui (40) disposée dans la direction axiale, au niveau du côté extérieur de la vis de retenue de clou, et une douille de raccordement (44) qui
- présente un moyen d'entraînement (68, 70) qui, dans l'état posé sur la vis de retenue de clou, coopère avec la surface d'appui (40) de sorte que la vis de retenue de clou tourne solidairement avec la douille de raccordement, et possède des moyens (52, 54, 56) pour bloquer en rotation la douille de raccordement par rapport au clou intramédullaire dans la position prédéfinie de la vis de retenue de clou.

2. Instrument de visée selon la revendication 1, **caractérisé en ce que** les joues (28, 30) de la vis de retenue de clou possèdent un filet (26) sur le côté extérieur en vue de la liaison avec un filet intérieur sur le clou intramédullaire.

3. Instrument de visée selon la revendication 1 ou 2, **caractérisé en ce que** le fond entre les joues est arrondi.

4. Instrument de visée selon une des revendications 1 à 3, **caractérisé en ce que** les extrémités libres des joues sont arrondies.

5. Instrument de visée selon une des revendications 1 à 4, **caractérisé en ce que** la vis de retenue de clou possède une saillie périphérique (36) sur l'extrémité dirigée vers le clou intramédullaire.

6. Instrument de visée selon la revendication 7, **caractérisé en ce que**, entre les joues et la saillie, il est prévu une rainure périphérique (34).

7. Instrument de visée selon une des revendications 1 à 6, **caractérisé en ce que** la douille de raccordement possède, sur le côté avant, trois saillies qui enferment par paire des angles de 90° ou 180°.

8. Instrument de visée selon une des revendications 1 à 7, **caractérisé en ce que** les moyens pour bloquer en rotation la douille de raccordement présentent, sur le côté avant, des saillies et/ou des creux.

9. Instrument de visée selon une des revendications 1 à 8, **caractérisé en ce que** le moyen d'entraînement de la douille de raccordement présente un alésage (68) et une broche (76) qui peut y être insérée.

10. Instrument de visée selon la revendication 9, **caractérisé en ce que** l'alésage (68) est disposé à distance d'un axe longitudinal médian de la douille de raccordement et **en ce que** la broche (76) appuie par sa surface d'enveloppe sur la surface d'appui.

11. Instrument de visée selon une des revendications 1 à 10, **caractérisé en ce que** la douille de raccordement possède des creux d'orientation (58, 60, 62) pour un segment de prise.

12. Instrument de visée selon la revendication 11, **caractérisé en ce que** le segment de prise possède des moyens de localisation dans le sens axial et dans le sens de rotation par rapport aux creux d'orientation.

13. Instrument de visée selon la revendication 12, **caractérisé en ce que** le segment de prise (88) possède un alésage pour loger la douille de raccordement, dans lequel une broche prétendue par ressort (98) fait saillie pour l'engagement dans un des creux d'orientation.

14. Instrument de visée selon la revendication 13, **caractérisé en ce que** le segment de prise possède une vis d'arrêt (102) qui, quand elle est en position vissée, presse la broche (98) dans un des creux d'orientation.

15. Instrument de visée selon une des revendications 1 à 14, **caractérisé en ce que** la vis de retenue de clou présente, au niveau de son extrémité opposée au clou intramédullaire, un segment fileté qui, quand la douille de raccordement est en place, fait saillie à partir de celle-ci.
